# Europäisches Patentamt
# European Patent Office
# Office européen des brevets

(19)

(11) Veröffentlichungsnummer: **0 075 218**
**B1**

(12) **EUROPÄISCHE PATENTSCHRIFT**

(45) Veröffentlichungstag der Patentschrift:
**12.09.84**

(51) Int. Cl.³: **C 07 C 11/06**, C 07 C 7/09,
**C 01 B 3/50**

(21) Anmeldenummer: **82108349.0**

(22) Anmeldetag: **10.09.82**

(54) Verfahren zur Abtrennung von Propylen und Synthesegas aus isobutyraldehydhaltigem Gemisch.

(30) Priorität: **19.09.81 DE 3137357**

(43) Veröffentlichungstag der Anmeldung:
**30.03.83 Patentblatt 83/13**

(45) Bekanntmachung des Hinweises auf die Patenterteilung:
**12.09.84 Patentblatt 84/37**

(84) Benannte Vertragsstaaten:
**DE FR GB IT**

(56) Entgegenhaltungen:
**DE - B - 1 917 244**

(73) Patentinhaber: **Ruhrchemie Aktiengesellschaft,
Bruchstrasse 219, D-4200 Oberhausen 13 (DE)**

(72) Erfinder: **Cornils, Boy, Dr. Dipl.-Chem., Friedrich-Ebert
Strasse 45, D-4220 Dinslaken (DE)**
Erfinder: **Konkol, Werner, Dr. Dipl.-Chem.,
Lützowstrasse 40A, D-4200 Oberhausen 13 (DE)**
Erfinder: **Diekhaus, Gerhard, Dr. Dipl.-Chem.,
Walsumermarkstrasse 89, D-4200 Oberhausen 14 (DE)**
Erfinder: **Wiebus, Ernst, Ferdinandstrasse 77,
D-4200 Oberhausen 14 (DE)**

(74) Vertreter: **Reichelt, Karl-Heinz, Dr., m. Br. Ruhrchemie
Aktiengesellschaft Abt. PLD Postfach 13 01 60,
D-4200 Oberhausen 13 (DE)**

## Beschreibung

Bei der grosstechnischen Hydroformylierung von Propylen wird in Abhängigkeit vom Katalysatorsystem und den übrigen Synthesebedingungen neben dem Wertprodukt n-Butyraldehyd stets Isobutyraldehyd als Nebenprodukt gebildet. Wegen der eingeschränkten technischen Verwendung von Isobutyraldehyd besteht ein berechtigtes Interesse, diesen ebenfalls in n-Butyraldehyd umzuwandeln. Nach einem bekannten Verfahren (vgl. DE-PS Nr. 1917244) wird das bei der Propylen-Hydroformylierung anfallende im wesentlichen aus n- und i-Butyraldehyd bestehende Gemisch zunächst getrennt, der abgetrennte Isobutyraldehyd katalytisch unter Bildung von Propylen, Wasserstoff und Kohlenmonoxid gespalten und das so entstandene Propylen-Synthesegas-Gemisch erneut der Hydroformylierung zugeführt.

Dieses Verfahren liefert bezogen auf das Einsatzprodukt Propylen höhere Ausbeuten an gewünschtem n-Butyraldehyd. Im Hinblick auf die willkürlich steigenden Rohstoffpreise kommt einem derartigen Verfahren eine zunehmend wachsende Bedeutung zu.

Die katalytische Spaltung von Isobutyraldehyd erfolgt im allgemeinen in der Gasphase, wobei Temperaturen von 250 bis 350°C und Drücke von 1 bis 10 bar angewendet werden. Als Katalysatoren werden in der Regel Festbettsysteme unterschiedlicher Zusammensetzung empfohlen. Die DE-PS Nr. 1917244 sieht den Einsatz eines Katalysators vor, der ca. 1% Rhodium auf Aluminiumoxid als Trägermaterial aufweist. Als Katalysatoren werden auch Rutheniumverbindungen (R.H. Prince und R.A. Raspin, „Chem. Comm." 1966, 156), Palladium [J. Tsuji und K. Ohno, „J. Amer. Chem. Soc." 90, 94 (1968)] oder Rhodiumverbindungen wie Chloro-tri-(triphenylphosphin)-rhodium [K. Ohno und J. Tsuji, „J. Amer. Chem. Soc." 90, S. 99-107 (1968)] zur Spaltung von Isobutyraldehyd in Propylen, Wasserstoff und Kohlenmonoxid eingesetzt.

Trotz der Möglichkeit, die Spaltung von Isobutyraldehyd in Propylen und Synthesegas bei einem sehr hohen Umsatz vorzunehmen, zieht man es in der Praxis häufig vor, diese Umsetzung nur mit einem Teilumsatz und gegebenenfalls in Gegenwart von Wasserdampf zu betreiben. Diese Verfahrensvariante besitzt den Vorteil, die Selektivität der Umsetzung zu verbessern und gleichzeitig die Bildung störender Nebenprodukte wirksam zu unterdrücken. Insbesondere führt die unerwünschte Bildung von Kohlenstoff zu einer sehr raschen Desaktivierung des Katalysators.

Das die Spaltungsvorrichtung nach Abkühlung auf Raumtemperatur verlassende gasförmige Reaktionsgemisch kann in Abhängigkeit von den gewählten Reaktionsbedingungen neben 10 bis 25 Gew.-% Isobutyraldehyd, 37 bis 50 Gew.-% Propylen, 2 bis 8 Gew.-% Propan, 29 bis 35 Gew.-% Kohlenmonoxid, 1,5 bis 2,5 Gew.-% Wasserstoff sowie Spuren an Isobutanol und Wasser aufweisen.

Eine komplette einstufige Auftrennung dieses Gemisches z.B. durch Druckdestillation oder Adsorptionsverfahren in Propylen und Synthesegas als Einsatzprodukt für die Hydroformylierung einerseits und nicht umgesetzten Isobutyraldehyd andererseits, der wieder der Spaltungsreaktion zugeführt wird, erfordert einen hohen technischen Aufwand. Da für den Wiedereinsatz von Propylen und Synthesegas in die Hydroformylierung eine Trennung von Synthesegas und Propylen nicht notwendig ist, genügt es, die gasförmigen Produkte vom nicht umgesetzten Isobutyraldehyd abzutrennen und auf den erforderlichen Druck von 150 bis 350 bar, wie er in der Hydroformylierung häufig angewendet wird, zu komprimieren.

Aus technischen Gründen ist es unumgänglich, die Kompression in mehreren Druckstufen vorzunehmen. Je höher der gewünschte Enddruck sein soll, um so mehr Druckstufen (Kompressionsschritte) sind erforderlich. Jeder dieser Druckstufen ist ein Kompressor und eine Druckvorlage zugeordnet. Aus der Druckvorlage erhält der nachfolgende Kompressor das jeweils anfallende Gasgemisch zugeführt. In der ersten Druckstufe wird ein Druck von 5 bis 15, in der zweiten ein solcher von 25 bis 75 bar und in der dritten der Enddruck von 150 bis 350 bar eingestellt.

Entsprechend dem jeweiligen Druck bildet sich in den Druckvorlagen jeweils eine flüssige Phase, die aus Propylen, Propan und restlichem Isobutyraldehyd besteht, und eine Gasphase, die ausser Propylen und Synthesegas im wesentlichen noch Propan sowie gegebenenfalls restlichen Isobutyraldehyd enthält, aus. Die flüssige Phase, die in jeder der zwei Druckvorlagen anfällt, wird jeweils in einen separaten Druckbehälter gebracht.

Lediglich in der ersten Vorlage, die einen Druck von 1 bis 10 bar aufweist, trennt sich Propylen von Isobutyraldehyd quantitativ.

In dem der zweiten Druckvorlage nachgeschalteten Druckbehälter hingegen erfolgt nur eine sehr unvollständige Trennung von Propylen, Propan und Synthesegas von dem noch vorhandenen Isobutyraldehyd.

Dies hat zur Folge, dass nahezu die Hälfte des insgesamt eingesetzten Propylens als Flüssigprodukt anfällt und wegen der starken Verunreinigung mit Isobutyraldehyd entweder in die Spaltungsreaktion zurückgefahren wird oder aber in einer separaten Druckdestillation zu reinigen ist.

Es bestand daher die Aufgabe, ein Verfahren zur Abtrennung von Synthesegas, Propan und Propylen aus einem Isobutyraldehyd haltigen Gemisch bereitzustellen, das diese Nachteile umgeht. Diese Aufgabe wird gelöst durch ein Verfahren, in dem das in den beiden Kompressionsstufen flüssig anfallende Produktgemisch aus den Druckvorlagen auf einen gemeinsamen Behälter, der bei Drücken von 1 bis 10 bar betrieben wird, abgeführt wird, wobei sich in der Vorlage Propylen, Propan und Synthesegas praktisch quantitativ abtrennen lassen und vor die erste Kompressionsstufe zurückgegeben werden, während flüssig anfallender Isobutyraldehyd wieder zur Spaltungsreaktion eingesetzt wird. Es zeigt sich überraschenderweise, dass es durch die beanspruchten Massnahmen gelingt, das gesamte Propylen aus dem bei der katalytischen Spaltung von Isobutyraldehyd anfallenden Gemisch abzutrennen, indem in der Sammelvorlage anfallendes Propylen im Kreis gefahren wird.

Nach der neuen Arbeitsweise wird das Einsatzgemisch, das unter einem Druck von 1 bis 10 bar anfällt, der Kompression unterworfen, wobei in der ersten Stufe ein Druck von 5 bis 15 bar und in der zweiten Stufe ein solcher von 25 bis 75 bar eingestellt wird. Jedem der beiden Kompressoren ist eine Druckvorlage, die nach dem Prinzip eines Gasabscheiders arbeitet, nachgeschaltet. Die gasförmig anfallenden Reaktionsprodukte aus der ersten Kompressionsstufe werden dem nachfolgenden Kompressor zugeführt, während die Flüssigprodukte aus beiden Druckvorlagen in einer gemeinsamen Sammelvorlage, die unter dem Druck des Einsatzgemisches von 1 bis 10 bar steht, vereinigt werden. Die Auftrennung von bei der katalytischen Spaltung nicht umgesetztem Isobutyraldehyd einerseits sowie Synthesegas, Propan und Propylen als Gasbestandteile andererseits erfolgt praktisch quantitativ, wobei die gasförmigen Komponenten über Kopf vor die Kompression zurückgeleitet werden. Nicht umgesetzter Isobutyraldehyd wird am Boden der Sammelvorlage, die ebenfalls nach dem Prinzip eines Gasabscheiders arbeitet, abgenommen und wieder der Isobutyraldehydspaltung zugeführt.

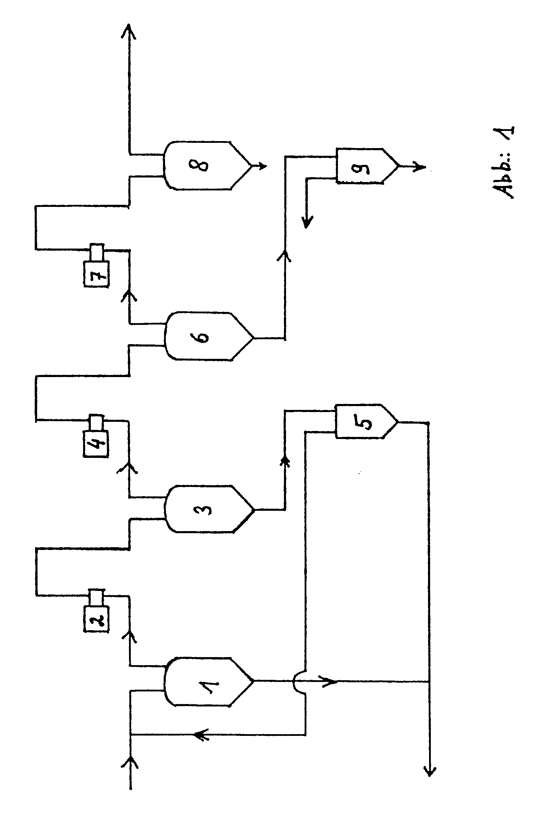

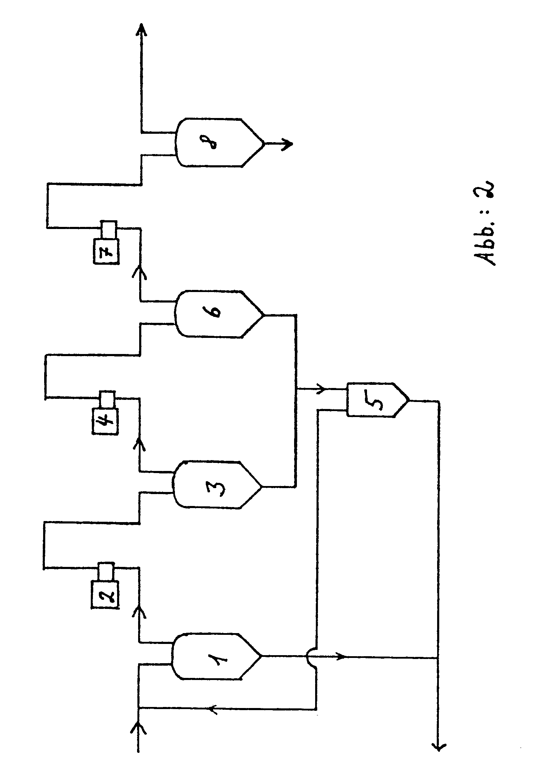

Das im folgenden angegebene Beispiel soll den Vorteil des erfindungsgemässen Verfahrens näher erläutern. In der Abb. 1 ist das Vergleichsverfahren und in der Abb. 2 das erfindungsgemässe Verfahren schematisch dargestellt. Hierbei bedeuten:

*Vergleichsverfahren*

1 : Einsatzvorlage
2 : Kompressor 1. Stufe
3 : Druckvorlage 1. Stufe
4 : Kompressor 2. Stufe
5 : Rückflussvorlage
6 : Druckvorlage 2. Stufe
7 : Kompressor 3. Stufe
8 : Druckvorlage 3. Stufe
9 : Propylenvorlage

*erfindungsgemässes Verfahren*

1 : Einsatzvorlage
2 : Kompressor 1. Stufe
3 : Druckvorlage 1. Stufe
4 : Kompressor 2. Stufe
5 : gemeinsame Sammelvorlage
6 : Druckvorlage 2. Stufe
7 : Kompressor 3. Stufe
8 : Druckvorlage 3. Stufe

Das die Spaltanlage (Spaltreaktor mit nachgeschaltetem Kondensator und Gasabscheider) unter einem Druck von 2 bar verlassende Gasgemisch (16 kg/h; 37,1 Gew.-% $C_3H_6$; 7,1 Gew.-% $C_3H_8$; 24,5 Gew.-% Isobutyraldehyd; 0,2 Gew.-% $H_2O$; 29,6 Gew.-% CO; 1,6 Gew.-% $H_2$) gelangt nach Vereinigung mit dem Gaskreislauf aus einer Rückflussvorlage (5) über eine Einsatzvorlage (1), an deren Sumpf geringe Mengen Kondensat (0,5 kg/h; 2,4 Gew.-% $C_3H_8$; 95,2 Gew.-% Isobutyraldehyd; 2,4 Gew.-% $H_2O$) abgenommen werden, zu Kompressor (2), wo der Druck auf 7 bar erhöht wird. Jede Kompressionsstufe ist mit einem Abscheider und einem vorgeschalteten Kühler (der nicht eingezeichnet ist) zur Abführung der Kompressionswärme ausgerüstet. Bei dem nicht beanspruchten Vergleichsverfahren gemäss Abb. 1 werden aus in der Druckvorlage (3) anfallenden Flüssigprodukt nach Entspannung auf 2 bar im Sumpf der Rückflussvorlage (5) 1,9 kg/h Flüssigprodukt ( 3 Gew.-% $C_3H_6$, $C_3H_8$, 96 Gew.-% Isobutyraldehyd, 1 Gew.-% $H_2O$) abgezogen. Nachdem der Druck des gasförmigen Gemisches vom Abscheider (3) mit dem Kompressor (4) von 7 bar auf 35 bar erhöht worden ist, werden aus dem im Abscheider (6) anfallenden Flüssigprodukt nach Entspannung auf 15 bar in der Propylenvorlage (9) 5,1 kg/h Flüssigpropylen (56,9 Gew.-% $C_3H_6$, 9,7 Gew.-% $C_3H_8$, 1,2 Gew.-% CO, 0,1 Gew.-% $H_2$, 32,1 Gew.-% Isobutyraldehyd) abgeschieden und 1,8 kg/h Abgas (32,3 Vol.% $C_3H_6$, 5,9 Vol.% $C_3H_8$, 35,8 Vol.% CO, 25,5 Vol.% $H_2$, 0,5 Vol.% Isobutyraldehyd) erhalten. Ausserdem fallen als gasförmiges Gemisch aus Abscheider (6) nach Kompression mit dem Kompressor (7) von 35 bar auf 290 bar 7,1 kg/h (16,1 Vol.% $C_3H_6$, 3,6 Vol.% $C_3H_8$, 44,4 Vol.% CO, 35,7 Vol.% $H_2$, 0,2 Vol.% Isobutyraldehyd) an.

Bei dem erfindungsgemässen Verfahren (Abb. 2) werden aus den im Abscheider (3) und im Abscheider (6) anfallenden Flüssigprodukten nach Entspannung auf 2 bar im Sumpf der Rückflussvorlage (5) 3,6 kg/h Flüssigprodukt (95,7 Gew.-% Isobutyraldehyd, 3,3 Gew.-% $C_3H_6$ + $C_3H_8$, 1% $H_2O$) abgezogen. Als gasförmiges Gemisch aus Abscheider (6) fallen nach Kompression mit dem Kompressor (7) von 35 bar auf 290 bar 12,2 kg/h (30 Vol.% $C_3H_6$, 5,4 Vol.% $C_3H_8$, 35,9 Vol.% CO, 28,5 Vol.% $H_2$, 0,2 Vol.% Isobutyraldehyd) an.

## Patentanspruch

Verfahren zur Abtrennung von Propylen und Synthesegas aus einem aldehydhaltigen Gemisch, das durch katalytische Spaltung von Isobutyraldehyd gebildet wird, durch stufenweise Kompression, dadurch gekennzeichnet, dass das aldehydhaltige Gasgemisch mit einem Druck von 1-10 bar in einer ersten Kompressionsstufe auf 5-15 bar und in einer zweiten Kompressionsstufe auf einen Druck von 25-75 bar gebracht wird, das komprimierte Produktgemisch in einer Druckvorlage in gasförmige und flüssige Produktbestandteile getrennt wird, wobei die Flüssigprodukte beider Druckvorlagen in einer gemeinsamen Sammelvorlage, die unter dem Druck des der ersten Kompressionsstufe zugeführten aldehydhaltigen Gasgemisches steht, vereinigt werden und dort der bei der Spaltung nicht umgesetzte Isobutyraldehyd quantitativ abgetrennt wird, indem die gasförmigen Bestandteile dem Einsatzmaterial zugeführt werden.

## Claim

Process for the separation by step-wise compression of propylene and synthesis gas from an aldehyde-containing mixture formed by catalytic splitting of isobutyraldehyd, characterised in that the aldehyde-containing gas mixture with a pressure of 1-10 bar is brought up to a pressure of 5-15 bar in an initial compression stage and to a pressure of 25-75 bar in a subsequent compression stage, the compressed product mixture is separated into gaseous and liquid product components in a pressure vessel whereby the liquid products of the two pressure vessels are combined in a common collecting vessel which is under the same pressure as the aldehyde-containing gas mixture led into the initial compression stage and where the isobutyraldehyde not converted during splitting is quantitatively separated by the gaseous components being fed into the feed material.

## Revendication

Procédé pour séparer du propylène et du gaz de synthèse d'un mélange contenant des aldéhydes, qui a été formé par scission catalytique de l'aldéhyde isobutyrique, par compression en plusieurs étages, caractérisé en ce que le mélange gazeux contenant des aldéhydes et à une pression de 1 à 10 bar est porté dans un premier étage de compression à un niveau de 5 à 15 bar et dans un deuxième étage de compression à une pression de 25 à 75 bar, le mélange de produits comprimé est séparé dans un réservoir sous pression en constituants de produits gazeux et constituants de produits liquides, les produits liquides des deux réservoirs sous pression étant combinés dans un réservoir de récolte commun qui est sous la pression du mélange gazeux contenant des aldéhydes envoyé au premier étage de compression, l'aldéhyde isobutyrique non converti à la scission étant séparé quantitativement à cet endroit et les constituants gazeux recyclés dans le produit de départ.

Abb.: 1

Abb.: 2